# EUROPÄISCHE PATENTSCHRIFT

(11) **EP 1 229 833 B1**
(45) Veröffentlichungstag und Bekanntmachung des Hinweises auf die Patenterteilung: **21.06.2006**
(21) Anmeldenummer: 00969476.1
(22) Anmeldetag: 10.10.2000
(51) Int. Cl.: A61B 5/083, A61B 5/097

(54) **ERGOSPIROMETRIESYSTEM FÜR TIERE, INSBESONDERE PFERDE ODER KAMELE**
ERGOSPIROMETRY SYSTEM FOR ANIMALS, ESPECIALLY HORSES OR CAMELS
SYSTEME ERGOSPIROMETRIQUE POUR ANIMAUX, NOTAMMENT POUR CHEVAUX OU CHAMEAUX

(30) Priorität: 16.11.1999 DE 19955121; 14.12.1999 DE 19960257
(43) Veröffentlichungstag der Anmeldung: 14.08.2002
(73) Patentinhaber: Cortex Biophysik GmbH, 04229 Leipzig (DE)
(72) Erfinder: GEHRKE, Matthias, 60318 Frankfurt/Main (DE); HENKER, Ralf, 04207 Leipzig (DE); KRETSCHMER, Claus-Peter, 04347 Leipzig (DE)
(74) Vertreter: Kruspig, Volkmar
(86) Internationale Anmeldenummer: PCT/EP2000/009965
(87) Internationale Veröffentlichungsnummer: WO 2001/047417

(56) Entgegenhaltungen:
- EP-A- 0 537 991
- WO-A-93/24810
- WO-A-98/53732
- WO-A-99/39637
- PATENT ABSTRACTS OF JAPAN vol. 018, no. 157 (C-1180), 16. März 1994 (1994-03-16) & JP 05 329132 A (ANIMA KK), 14. Dezember 1993 (1993-12-14)

## Beschreibung

Die Erfindung betrifft ein Ergospirometriesystem für Tiere, insbesondere Pferde oder Kamele, umfassend eine trichterförmige, zylindrische oder halbkugelförmige Atemgasmaske, Gasvolumenstrom- oder Mengensensor sowie eine Meßeinheit mit Sensoren zur Bestimmung der CO₂/O₂-Konzentration im Atemgas nach dem Mischkammer-oder Breath-by-Breath-Prinzip und Anschluß- und/oder Signalübertragungsmittel zur Meßwert-Weiterverarbeitung, -Darstellung und/oder -Analyse in einer Basisstation.

Mobile Ergospirometriegeräte, z.B. für Belastungsuntersuchungen beim Menschen außerhalb des Labors sind seit einigen Jahren bekannt. Mit Hilfe derartiger mobiler Systeme lassen sich Analysen direkt auf dem Sport- oder am Arbeitsplatz unter natürlichen Bedingungen und Belastungssituationen durchführen. Über Telemetrieeinheiten werden die Meßdaten in Echtzeit an einen Personal-Computer oder ein Notebook übertragen, wobei eine entsprechende Steuerung des Trainings- oder Übungsverlaufs nach Auswertung der Daten möglich ist. Durch derartige Geräte wurden neue Anwendungsgebiete in der Leistungsdiagnostik, in Arbeits-, Sport- und der Rehabilitationsmedizin erschlossen.

Die WO 99/39637 offenbart als nächstliegenden Stand der Technik ein Ergospirometriesystem mit einer trichterförmigen Atemgasmaske und mit einem Gasvolumenstromsensor mit einem von der Maske lösbaren Volumenstromsensorgehäuse, wobei an der Atemmaske Mittel zum Befestigen des Volumenstromsensorgehäuses vorgesehen sind, das Volumenstromsensorgehäuse eine Strömungskammer aufweist, die Strömungskammer jeweils Gaseinlaß- bzw. Gasauslaßöffnungen umfaßt, welche einerseits in Strömungskontakt mit einer zugehörigen Öffnung der Maske und andererseits zur Umgebung stehen, und Ultraschallwandler zur Bestimmung des Volumenstroms vorgesehen sind.

Aus der WO 98/53732 ist ein tragbares Ergospirometriesystem mit einer Telemetrie-Datenübertragungseinheit bekannt. Mit der dort gezeigten Anordnung sollen individuelle Parameter hinsichtlich der Sauerstoffaufnahme und der Kohlendioxidproduktion erfaßbar, d.h. eine Atemanalyse in Verbindung mit der Bestimmung des Herzrhythmus unter natürlichen Umgebungsbedingungen des Menschen möglich sein.

Gemäß der bekannten Lehre besitzt eine tragbare Einheit, die am Probanden befestigt ist, neben einer Atemmaske eine Gasanalyse-Einrichtung zur Bestimmung der O₂- und CO₂-Werte des Meßgases.

Weiterhin ist dort ein Herzfrequenz-Monitor vorgesehen, wobei die Meßwerte einem Mikroprozessor zugeführt werden, der Bestandteil der tragbaren Einheit ist. Die Meßwerte werden in einem internen Speicher abgelegt und über eine Telemetriestrecke zu einem Telemetrieempfänger übertragen, der wiederum mit einem Personal-Computer-System in Verbindung steht.

Mobile Ergospirometriesysteme und die dort vorhandene Technik insbesondere zur Bestimmung des Volumenstroms bei menschlichen Probanden lassen sich nicht ohne weiteres für veterinärdiagnostische Zwecke anwenden oder übertragen.

Beispielsweise verändert sich bei Pferden die Atemzugsanzahl pro Minute ausgehend von einem Ruhewert im Bereich von 10 bis 14 bei Belastung auf bis maximal 120 bis 150 Atemzüge mit der Folge erheblicher, variierender Volumenströme. Darüber hinaus tragen Tiere einen wesentlich höheren Feuchteanteil aus, wodurch eingesetzte Sensoren bedingt durch Feuchteablagerungen beschädigt oder in der Funktion beeinträchtigt werden können, mit der Folge unzureichender Meßergebnisse oder mangelnder Zuverlässigkeit derartiger Geräte.

Weiterhin kann bei Anwendung des Mischkammer-Prinzips eine notwendige Pumpe für den Pneumatikkreislauf der in der Regel viel höheren Atemfrequenz der Tiere nicht oder nicht ausreichend folgen, wobei zusätzlich die Gefahr besteht, daß der im Absaugschlauch, welcher zwischen Volumenstromsensor und Pumpe angeordnet ist, entstehende Unterdruck wegen der Atemfrequenz und der größeren Länge kritische Werte erreicht, wobei hier die Gefahr besteht, daß nach Beendigung der Expirationsphase Umgebungsluft angesaugt wird und eine Meßwerteverfälschung eintritt.

Aus dem Vorgenannten ist es daher Aufgabe der Erfindung, ein Ergospirometriesystem für Tiere, insbesondere Pferde, Kamele oder dergleichen anzugeben, welches von einer Meßeinheit mit Sensoren zur Bestimmung der CO₂/O₂-Konzentration im Atemgas und einem speziellen Gasvolumenstrom- oder Mengensensor ausgeht, wobei weiterhin Anschluß- und/oder Signalübertragungsmittel zur Meßwert-Weiterverarbeitung, -Darstellung und/oder -Analyse vorgesehen sind. Bei dem zu schaffenden Ergospirometriesystem gilt es, bedingt durch die speziellen Gegebenheiten hinsichtlich insbesondere der Atemfrequenz und des notwendigen Abstands zwischen der Anordnung eines Volumenstromsensors und der Meßeinheit mit den Sensoren zur Bestimmung der CO₂/O₂-Konzentration eine Lösung zu finden, die auch dann für exakte Meßwerte Sorge trägt, wenn Nässe und Verschmutzung, die vom Tier herrühren, beim Atmen in einen Strömungskörper gelangen, welcher den Volumenstromsensor aufweist, oder wenn derartige Verschmutzungen beim Einatmen aufgenommen werden.
Insbesondere bei Anwendung des Mischkammer-Prinzips soll erfindungsgemäß sichergestellt werden, daß nicht unerwünscht Umgebungsluft angesaugt wird oder daß ein per se eine gewisse Trägheit aufweisendes Pneumatiksystem zu Meßwert-Verfälschungen führt, wenn die Atemfrequenz bei Belastung des Tieres erhöht ist.

Der zum Ergospirometriesystem gehörende Volumenstromsensor soll demnach geeignet sein, unter realen Umweltbedingungen sowohl in Ruhestellung als auch bei Bewegung des Tieres auf einem Laufband oder natürlicher Bewegung über kurze und längere Zeiten exakte Meßwerte zu liefern.

Die Lösung der Aufgabe der Erfindung erfolgt mit einem Ergospirometriesystem gemäß den Merkmalen des Patentanspruchs 1, wobei die Unteransprüche mindestens zweckmäßige Ausgestaltungen und Weiterbildungen, insbesondere mit Blick auf die Pneumatiksteuerung für ein Mischkammer-Meßprinzip umfassen.

Demgemäß besteht der Grundgedanke der Erfindung darin, bei dem Ergospirometriesystem für Tiere an der Atemgasmaske Mittel zum Befestigen eines von der Maske lösbaren Volumenstromsensorgehäuses vorzusehen. Diese Befestigungsmittel können form- und/oder kraftschlüssig, z.B. nach dem Snap-in-Prinzip ausgebildet sein, so daß vor Ort zunächst in einfacher Weise die Maske am Tier fixiert und abgedichtet werden kann, um dann in einem nächsten Schritt das Sensorgehäuse mit seinen elektrischen bzw. pneumatischen Anschlüssen an der Maske sicher zu fixieren.
Auch ohne Wechsel der Atemgasmaske soll ein schneller und einfacher Austausch des Volumenstromsensorgehäuses entsprechend den jeweiligen Versuchsbedingungen möglich sein.

Das vorgestellte Volumenstromsensorgehäuse weist mindestens eine Strömungskammer auf, wobei die Strömungskammer jeweils Öffnungen umfaßt, die einerseits im Strömungskontakt mit der oder den zugehörigen Öffnungen der Maske und andererseits zur Umgebung hin stehen.

Innerhalb der Strömungskammern werden durch in Strömungsrichtung verlaufende, parallel und beabstandet angeordnete Blenden Strömungskanäle gebildet. Die Blenden besitzen versetzt ausgebildete Öffnungen vorgebbaren Querschnitts, bevorzugt kreisrund oder elliptisch. Die Schwer- oder Mittelpunkte der Öffnungen verlaufen im wesentlichen auf einer gedachten Verbindungslinie, die sich wiederum bevorzugt diagonal in der jeweiligen Kammer ausrichtet.

An den Endpunkten der gedachten Linie sind Ultraschallwandler zur Bestimmung des Volumenstroms befindlich, wobei die Schallkeule der Wandler im wesentlichen entlang der Verbindungslinie verläuft.

Die Wandler sind jeweils abwechselnd in einer Betriebsart als Sender oder Empfänger geschaltet, wodurch der Einfluß von Störgrößen gemindert und Meßfehler eliminiert werden können.

Mit Hilfe der die Strömungskanäle bildenden Blenden wird das vorbeiströmende Atemgas gleichgerichtet bzw. homogenisiert, bleibt aber turbulent, ohne daß unerwünschte Verwirbelungen auftreten. Weiterhin dienen die Blenden mit ihren Öffnungen einer gezielten Beeinflussung der Ausbreitung des Ultraschallsignals mit der Folge, daß unerwünschte Reflexionen unterdrückt bzw. ausgeblendet werden. Durch Steuerung des zeitlichen Verlaufs der Ultraschallsignale und eine hierauf abgestellte Signalanalyse verbessert sich die Aussagegenauigkeit der Volumenstrom-Informationen, welche eine Voraussetzung für die objektive Meßwerterfassung sind.

Durch Auswahl der Ultraschallfrequenz und Aufbringen einer widerstandsfähigen, schmutz- und wasserabweisenden Oberflächenbeschichtung der Wandler gelingt es, ausreichend hohe Schallenergie zu erzeugen bzw. die Selektivität der Wandler zu sichern, so daß unweigerliche Veschmutzungen auf den Ultraschallwandlern das Meßergebnis nicht oder nur unwesentlich verfälschen. Beispielsweise kann eine solche Beschichtung aus Polytetrafluorethylen, einem partiell aromatischen Polyamid oder einem ähnlichen Material bestehen. Die Arbeitsfrequenz der Wandler liegt im Bereich von im wesentlichen 350 bis 500 kHz, vorzugsweise bei 400 bis 450 kHz.

Die Atemgasmaske ist erfindungsgemäß derart abgedichtet am Kopf des Tieres anbringbar, daß der Atemgasstrom ausschließlich zu und über den im Volumenstromsensorgehäuse vorgesehenen Strömungskammern führt, wobei zwischen dem Volumenstromsensorgehäuse und der Maske weitere Dichtmittel oder Dichtflächen vorgesehen sein können.

Die Ultraschallwandler sind mit dem umgebenden Gehäuse des Volumenstromsensors stoff- und/oder formschlüssig verbunden, wobei bevorzugt die eingesetzten Wandler eine gering dimensionierte Wandlerfläche mit einer Strahlungskeule kleinen Öffnungswinkels aufweisen.

Die Blenden innerhalb der Strömungskammern besitzen eine geringe, strömungsoptimierte Dicke und eine weitgehend ebene, glatte Oberfläche. Bevorzugt kommt Edelstahlmaterial mit einer Breite im Bereich von 0,15 bis 0,45 mm, bevorzugt 0,2 bis 0,4 mm zum Einsatz.

Innerhalb des Volumenstromsensorgehäuses ist eine Signalvorverarbeitungs-Elektronik zur Ansteuerung der Wandler und zur Bestimmung des Volumenflusses angeordnet, wobei die derartig vorverarbeiten, nunmehr unkritischeren Signale über eine entsprechende elektrische Verbindung zur Meßeinheit übertragbar sind.

Am Volumenstromsensorgehäuse ist ein Gasabsaug-Anschlußstutzen zur Herstellung einer Verbindung mit dem Zweck des Gastransports in die abgesetzt angeordnete Meßeinheit vorgesehen.

Das Volumenstromsensorgehäuse selbst kann aus Kunststoff gefertigt sein und besitzt eine im wesentlichen symmetrische Grundform mit zwei benachbarten Strömungskammern, wobei die Strömungskammern sich in verlängerter Richtung der Öffnungen in der Atemgasmaske, bevorzugt im Bereich der Nüstern des Tieres erstrecken. Der zwischen den Strömungskammern verbleibende Raum kann zur Aufnahme der Signalvorverarbeitungs-Elektronik ausgebildet sein.

In jeder Strömungskammer sind Ultraschallwandler angeordnet, wobei durch die zugehörige Auswerteelektronik die Gasvolumina bzw. der Volumenfluß je Kammer und der Summenfluß ermittelbar sind bzw. ist.

Die am Körper des Tieres oder am Körper einer führenden Person angeordnete Meßeinheit mit CO₂/O₂-Sensoren besitzt ein Telemetriemodul, wobei die Basisstation, welche entfernt angeordnet ist, eine Telemetrieeinheit zum Aufbau einer uni- oder bidirektionalen Daten- und/oder Befehlsübertragungsstrecke aufweist.

Mit Hilfe der Telemetrie besteht die Möglichkeit, Online-Daten zu erfassen, diese zu verarbeiten, mindestens teilweise auszuwerten, aber auch gleichzeitig Steuersignale zur Meßeinheit bzw. zur Führungsperson zu senden.

Die Telemetrieeinheit der Basisstation kann weiterhin eine automatische Frequenzwahl-Einrichtung aufweisen, um nach Scannen des relevanten Frequenzbands und/oder nach Ablauf einer Testdaten-Übertragungsprozedur eine Telemetriefrequenz mit erwarteter optimaler Übertragungsqualität festzulegen bzw. auszuwählen.

Durch einen Speicher zur Datensicherung, welcher in der Meßeinheit befindlich ist, besteht die Möglichkeit, in diesem Speicher Datensätze im definierten Format aus atemzugsbezogenen Meßdaten für jeden Atemzug und/oder für sämtliche oder ausgewählte Meßgrößen die kompletten Meßdatenverläufe über wählbare Intervalle zu wählbaren Startzeiten abzulegen.

Ergänzend können in der Meßeinheit Sensoren zur Bestimmung der Umgebungstemperatur und/oder der Atemgasfeuchte und/oder weiterer relevanter Größen, wie z.B. Differenzdruckwerte, vorgesehen sein, wobei diese Sensorwerte z.B. zur Kalibrierung und Korrektur der Atemgas-Sensorwerte herangezogen werden können.

Bei einer Ausführungsform der Erfindung ist in der Meßeinheit eine Mischkammer mit einer Pneumatiksteuerung vorhanden, wobei an der Mischkammer die CO₂/O₂-Sensoren, mit dieser in Strömungsverbindung stehend, angeschlossen sind.
Weiterhin ist bei dieser Ausführungsform eine Pumpe mit einem Absaugschlauch vorgesehen, wobei der Absaugschlauch zum Volumenstromsensorgehäuse bzw. zum dort vorgesehenen Gasabsaug-Anschlußstutzen führt und wobei über die Pneumatiksteuerung zwei Strömungskreisläufe für Expiration und Inspiration schaltbar sind.

Die in der Meßeinheit vorgesehene Pumpe arbeitet erfindungsgemäß quasi im Dauerbetrieb, wobei während der Inspiration die Pumpe mit einem Ruhewert einen Bypass-Strömungskreislauf versorgt, wodurch ein Eindringen von Umgebungsluft in die Mischkammer und/oder den Absaugschlauch wirksam verhinderbar ist.
Bei Expiration wird die Pumpe volumenstromgesteuert und versorgt die Mischkammer mit Atemgas, wobei während der Übergangsphase von Expiration zu Inspiration der Druckausgleich über eine durch die Pneumatiksteuerung zeitweise geschaltete Verbindung zwischen Mischkammer und Pumpeneingang erfolgt, wodurch das Eindringen von Umgebungsluft in den Absaugschlauch vermieden werden kann.

Durch die kontinuierliche Arbeitsweise der Pumpe, während der Inspiration mit einem definierten Ruhewert und bei Expiration gesteuert durch den Volumenstrom, ist keine Beschleunigung der Pumpe aus dem quasi Stillstand erforderlich, so daß auch eine entsprechend hohe Atmungsfrequenz, wie bei Tieren üblich, die Meßwerterfassung nicht beeinträchtigt.

Die Meßeinheit kann weiterhin Anzeige- und Bedienurigsmittel aufweisen, wodurch bestimmte Meßwerterfassungs- und -auswertemodi gewählt werden können und insbesondere durch eine das Tier führende Person eine erste Auswertung der Ergebnisse vorgenommen werden kann.
Die bevorzugt über eine Telemetrieeinheit mit Daten versorgte Basisstation ist durch einen üblichen Personal-Computer mit entsprechender Ergospirometrie-Software zur Meßwert-Verarbeitung und -Analyse realisierbar. Es ist jedoch bevorzugt vorgesehen, daß die Meßeinheit sämtliche Funktionseinheiten eines Ergospirometriesystems einschließlich Berechnung und Speicherung der Meßwerte sowie Steuerungseinheiten und Kommunikationsfunktionen aufweist bzw. enthält. Damit soll eine Bewertung von Gasanalysen z.B. bei Bewegung des Tieres im Gelände respektive natürlicher Umgebung möglich sein.

Durch eine entsprechende Verkabelung einschließlich Absaugschlauch besteht im stationären Betrieb die Möglichkeit, die Meßeinheit neben dem zu untersuchenden Tier aufzustellen, um störende Einflüsse zu vermeiden.

Die Anordnung der Ultraschallwandler innerhalb der Strömungskammern bzw. der Strömungskanäle ermöglicht einen direkten unmittelbaren Kontakt mit dem vorbeiströmenden Gas, wobei durch die geometrische Gestaltung ein Selbstreinigungs- und Selbsttrocknungseffekt erreicht wird. Vorgesehene Blenden mit kreisrunden und/oder elliptischen Durchbrüchen optimieren die Ausbreitung des Ultraschallsignals und verbessern die Störsicherheit insbesondere mit Blick auf mögliche Reflexionen des Ultraschallsignals im Strömungskanal.

Das Abdichten der Atemgasmaske am Kopf des Tieres erfolgt bevorzugt durch einen integral ausgebildeten Dichtkragen, der durch Beaufschlagen mit Luft sich der Oberflächenform des entsprechenden Bereichs des Tieres anpaßt und diesen dicht umschließt, ohne daß sich die Trageeigenschaften der Maske verschlechtern oder ein umständliches Handling beim Anlegen der Maske gegeben ist. Die Maske selbst kann zusätzlich durch ein Geschirr am Kopf des Tieres fixiert werden.

Alles in allem gelingt es mit der vorstehend beschriebenen Erfindung, ein neuartiges Ergospirometriesystem für Tiere anzugeben, das den besonderen Bedingungen, wie hohe Atmungsfrequenz bei Belastung, hohen Feuchtegrad des Atemgases sowie möglichen Verschmutzungen der Volumenstromsensoren genügt. Das System selbst ermöglicht auch einen längeren, störungsfreien Betrieb, wodurch Messungen bei ganz unterschiedlichen, aufeinanderfolgenden Belastungs- und Ruhephasen vorgenommen werden können.

Die Erfindung soll nachstehend anhand der Beschreibung eines Ausführungsbeispiels sowie unter Zuhilfenahme von Figuren näher erläutert werden.

Hierbei zeigen:
- Fig. 1: eine prinzipielle Darstellung einer am Tier befestigten Atemgasmaske mit fixiertem Volumenstromsensorgehäuse;
- Fig. 2: eine Darstellung der Atemgasmaske mit integriertem Dichtkragen;
- Fig. 3: eine Darstellung des einsatzbereiten Volumenstromsensorgehäuses mit Absaugschlauch und elektrischer Verkabelung;
- Fig. 4: eine Detaildarstellung des Volumenstromsensorgehäuses mit erkennbaren Strömungskammern sowie dem Anschluß für den Absaugschlauch;
- Fig. 5: verschiedene Ansichten des an der Maske fixierten Volumenstromsensorgehäuses;
- Fig. 6: den Systemaufbau aus Meßeinheit, Atemgasmaske und Volumenstromsensorgehäuse;
- Fig. 7: Vorderansicht und Schnittdarstellung des Volumenstromsensorgehäuses mit erkennbaren Strömungskammern und dort ausgebildeten Strömungskanälen einschließlich der vorgesehenen Ultraschallwandler;
- Fig. 8: Anordnungsbeispiele der Ultraschallwandler in der jeweiligen Strömungskammer bzw. dem Strömungkanal;
- Fig. 9: eine prinzipielle Darstellung der Öffnungen in den Blenden mit gedachter Verbindungslinie, an deren Enden die Ultraschallwandler innerhalb des Gehäuses angeordnet sind; und
- Fig. 10: ein Pneumatikschema mit Pumpe und Mischkammer.

Beim Ausführungsbeispiel wird von einem Ergospirometriesystem für Tiere, insbesondere für Pferde ausgegangen.

Eine Atemgasmaske 1 wird über den vorderen Teil des Tierkopfs gestülpt und dort durch ein Geschirr 2 befestigt.

Die Atemgasmaske ist so dicht am Tierkopf befestigt, daß Atemgas nur durch vorgesehene Öffnungen ein- bzw. ausströmen kann.

Ein Volumenstromsensorgehäuse 3 ist mit Befestigungsmitteln 4 an der Atemgasmaske 1 fixiert.

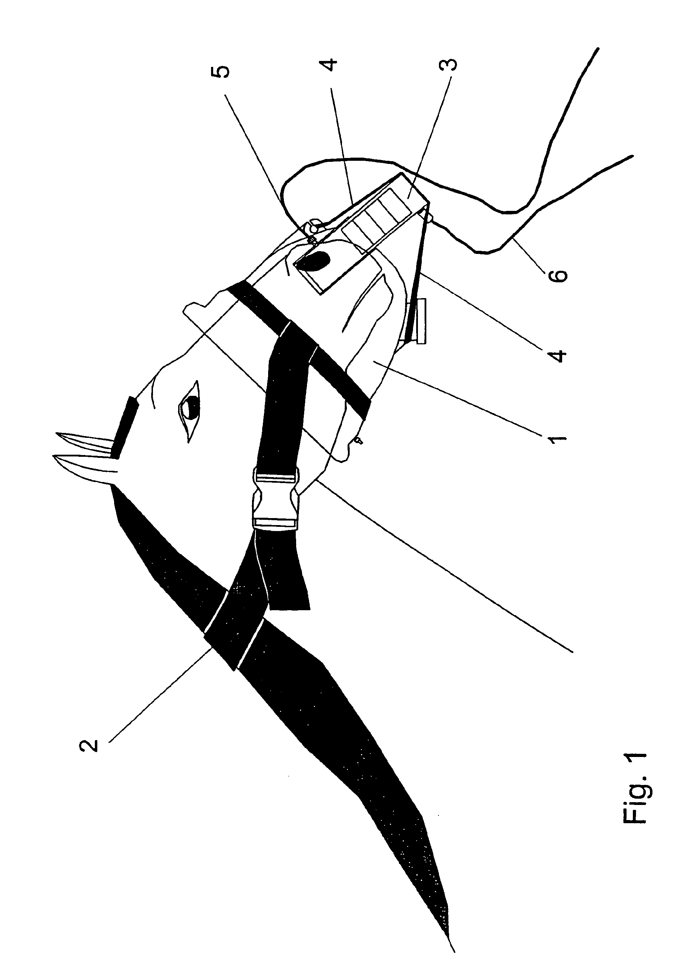

Über einen entsprechenden Anschlußstutzen ist ein Gasabsaugschlauch 5 im oberen Bereich des Volumenstromsensorgehäuses 3 befestigt, wobei dieser zur in Fig. 1 nicht dargestellten Meßeinheit führt. Gleiches gilt für die elektrischen Anschlüsse 6 der im Volumenstromsensorgehäuse 3 befindlichen Signalvorverarbeitungs-Elektronik. Dadurch, daß der Absaugschlauch 5 im oberen Teil des Volumenstromsensorgehäuses 3 angebracht ist, wird ein unerwünschtes Eindringen von Feuchtigkeit vermieden.

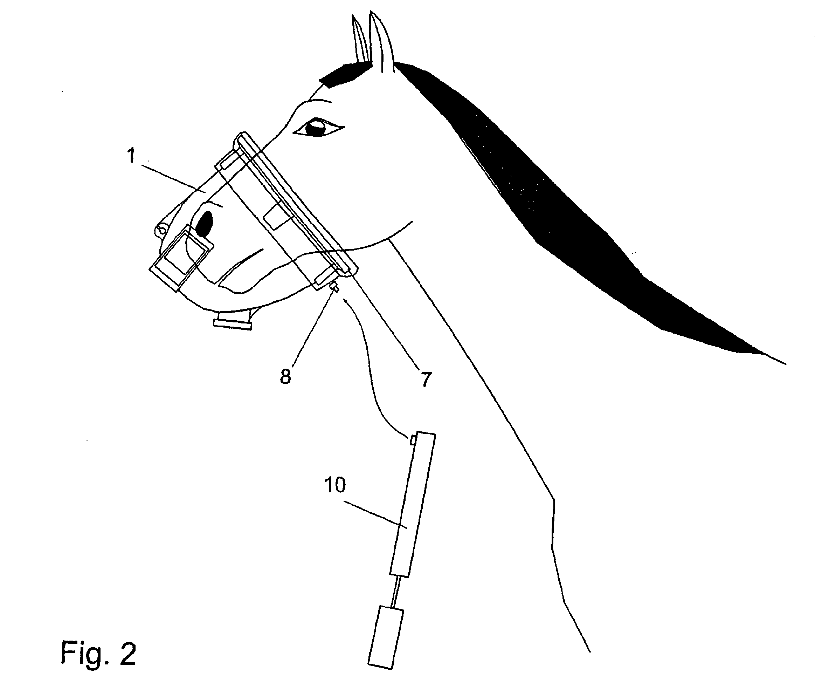

Fig. 2 macht deutlich, wie die Atemgasmaske 1 abdichtend fixierbar ist. Hierfür ist in der Atemgasmaske 1 ein integraler, aufblasbarer Dichtkragen 7 befindlich, der über ein Ventil 8 mit Druckluft beaufschlagt werden kann.
Dies geschieht mit einer handelsüblichen Luftpumpe 10 in besonders einfacher Weise.

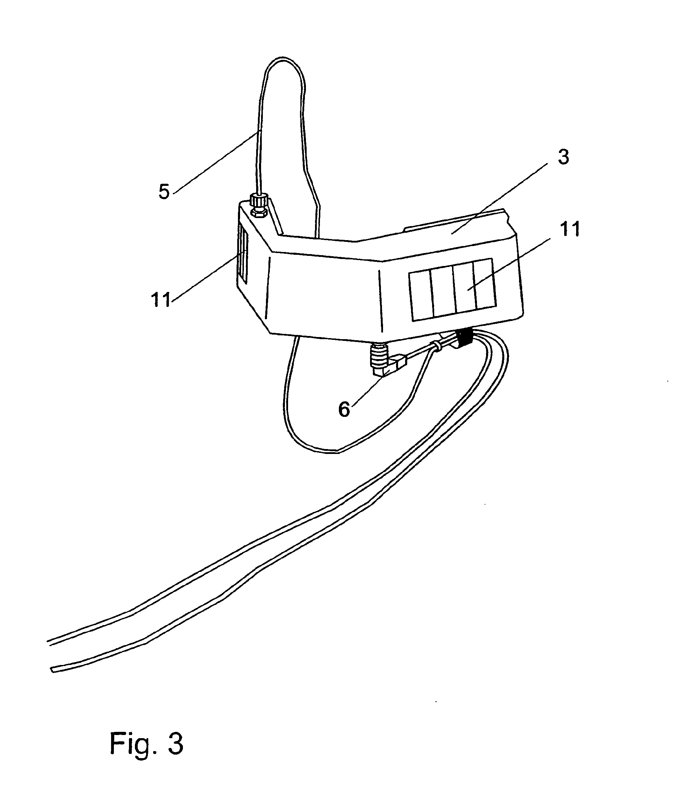

Fig. 3 läßt das Volumenstromsensorgehäuse 3 mit den bereits erwähnten elektrischen Anschlüssen 6 sowie dem Absaugschlauch 5 erkennen. Gleichfalls sind die Gasauslaß-Einlaßöffnungen 11 dargestellt.

Es liegt im Sinne der Erfindung, daß das System aus Atemgasmaske mit dort befestigtem Volumenstromsensorgehäuse mittels Kalibrierkammer oder Kalibrationspumpe vor dem Anbringen am Kopf des Tieres kalibriert bzw. geeicht werden kann. Damit wird Meß- und Versuchszeit eingespart und eine höhere Meßgenauigkeit gewährleistet.

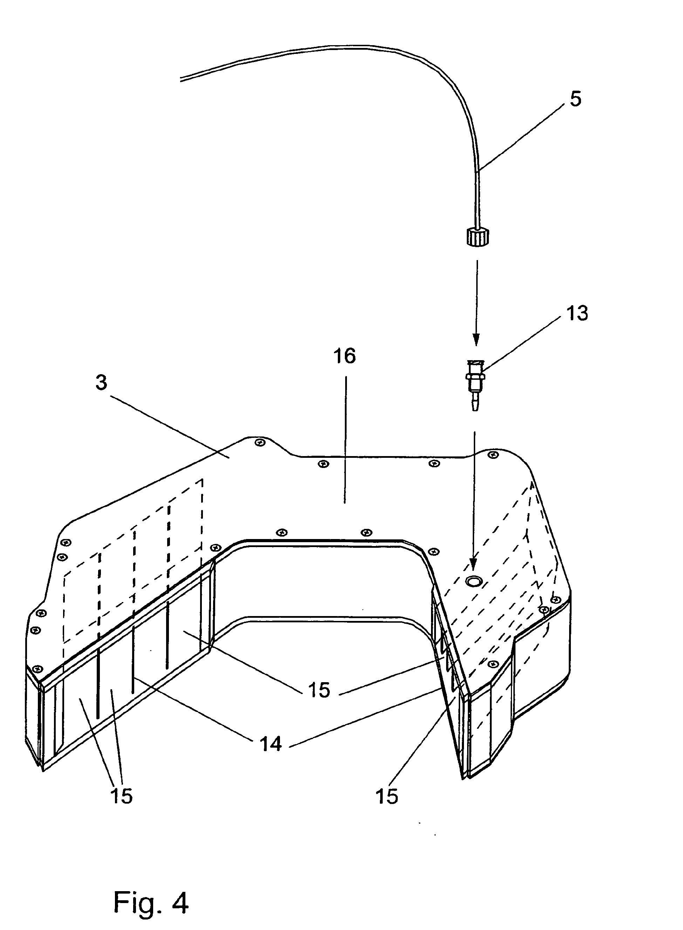

Der bereits erwähnte Anschlußstutzen 13 für den Gasabsaugschlauch ist in Fig. 4 besonders deutlich zu erkennen. Gleichfalls zeigt Fig. 4 die Strömungskammern 14 einschließlich der in den Kammern ausgebildeten Strömungskanäle 15.

Im mittleren, zwischen den Strömungskammern 14 liegenden Bereich 16 ist die Signalvorverarbeitungs-Elektronik anordenbar.

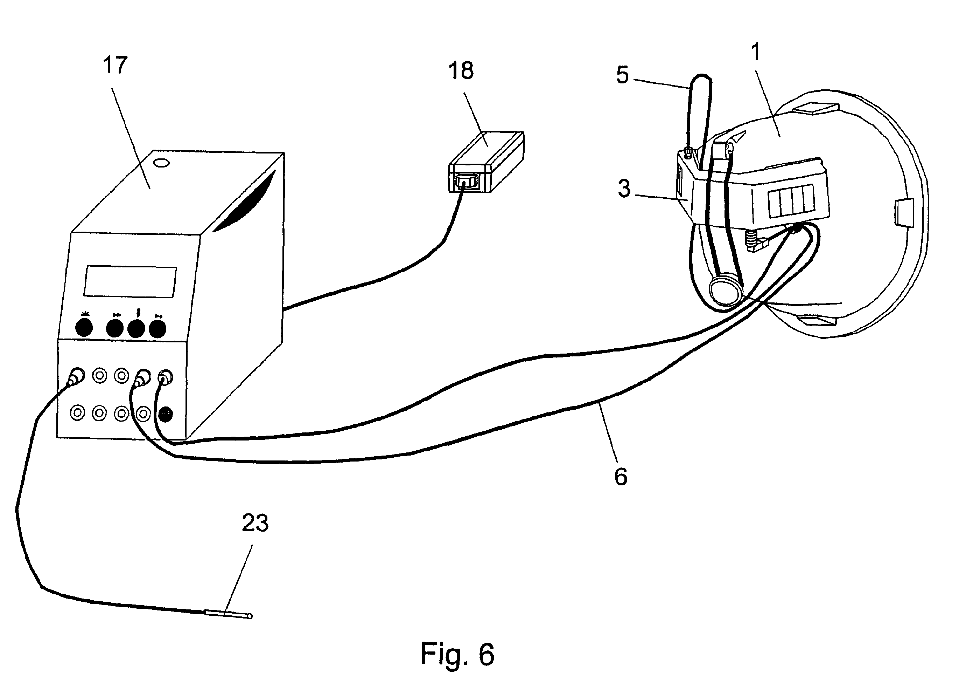

Der prinzipielle Systemaufbau gemäß Fig. 6 besteht also in einer Meßeinheit 17, die die Sensoren zur Bestimmung der CO₂/O₂-Konzentration im Atemgas und entsprechende Speicher- und Recheneinheiten sowie Bedien- und Anzeigeelemente aufweist. Die Meßeinheit 17 wird über Batterie-Pack 18 bzw. einem Netzteil mit Elektroenergie versorgt.
Die Meßeinheit 17 kann sowohl am Körper des Tieres befestigt werden, als auch durch eine das Tier führende Person betätigt bzw. von dieser getragen werden. Über entsprechend ausgebildete Längen der Verbindungsmittel Absaugschlauch und Elektroanschluß besteht die Möglichkeit der Anordnung der Meßeinheit neben dem Tier, z.B. wenn diese sich auf einem Laufband bewegt.

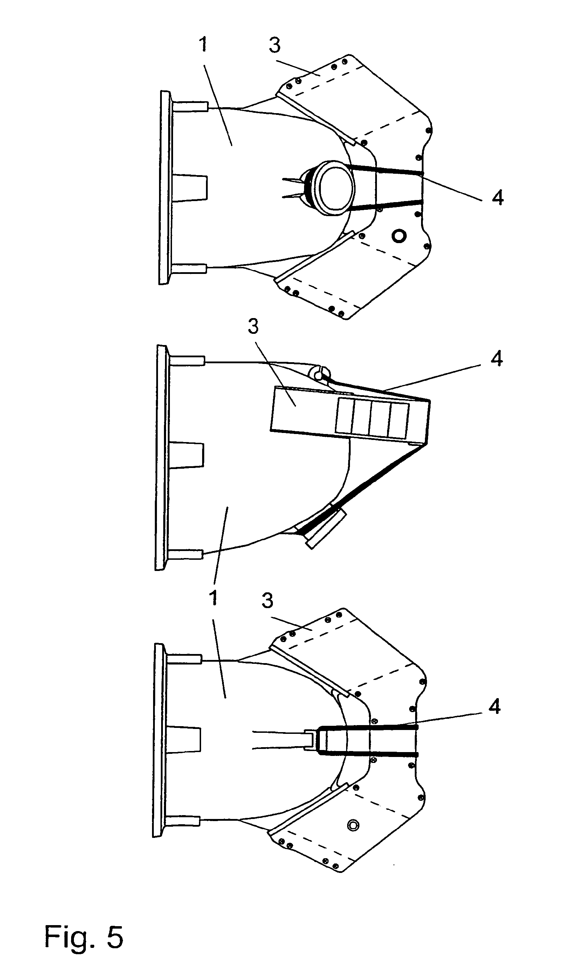

Die Ansichten nach Fig. 5 lassen die Befestigung des Volumenstromsensorgehäuses 3 an der Atemgasmaske 1 deutlich werden. Im oberen Teil ist eine Unteransicht, im mittleren Teil eine Seitenansicht und im unteren Teil der Fig. 5 eine Draufsicht dargestellt.
Bei der Darstellung nach Fig. 5 wird von einer gummibandunterstützten Preßverbindung zwischen Atemgasmaske 1 und Volumenstromsensorgehäuse 3 ausgegangen, wobei jedoch auch eine Snap-in-Verbindung realisierbar ist.

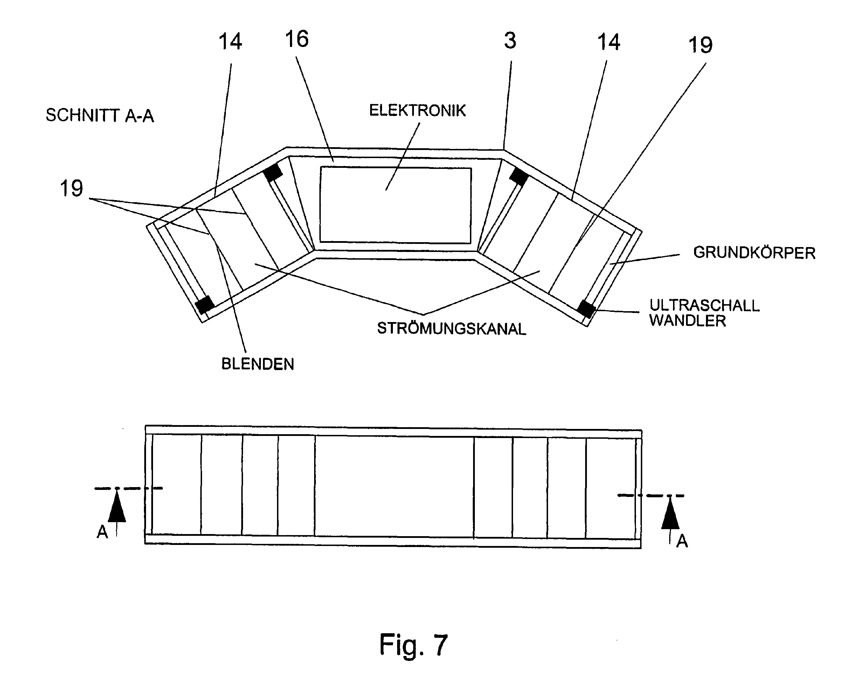

Der prinzipielle Aufbau des Volumenstromsensorgehäuses mit Strömungskammern und Strömungskanälen soll anhand der Fig. 7 bis 9 nachstehend näher erläutert werden.

Innerhalb des Volumenstromsensorgehäuses 3 befinden sich zwei Strömungskammern 14, die durch Blenden 19 jeweils in einzelne Strömungkanäle unterteilt sind.

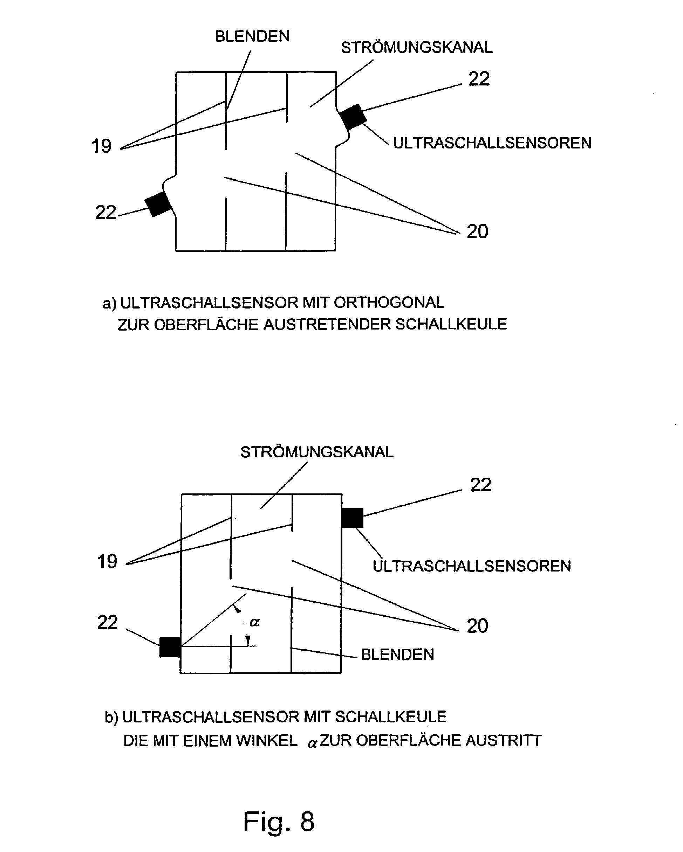

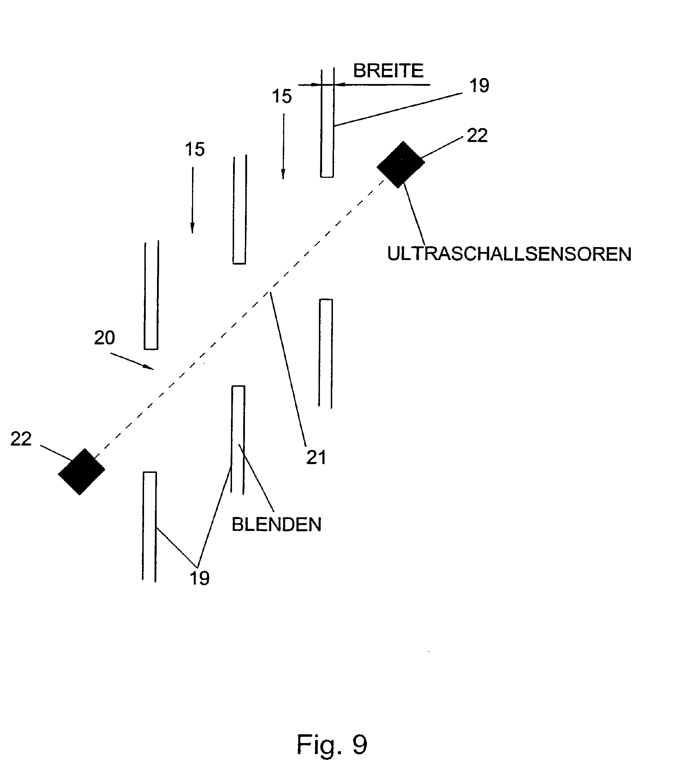

Die Blenden 19 sind in Strömungsrichtung verlaufend parallel und nahezu gleichmäßig beabstandet angeordnet, wobei in den Blenden 19 versetzt ausgebildete Öffnungen 20 (siehe Fig. 8 und 9) eingebracht sind. Diese Öffnungen 20 können z.B. einen kreisrunden, aber auch einen elliptischen Querschnitt aufweisen.

Die Öffnungen 20 liegen auf einer gedachten Verbindungslinie 21 der Schwer- oder Mittelpunkte, wobei an den Endpunkten der gedachten Linie 21 (siehe Fig. 9) Ultraschallwandler 22 befindlich sind. Die Schallkeule der Ultraschallwandler 22 erstreckt sich im wesentlichen entlang der gedachten Linie 21 und besitzt einen recht schmalen Austritts- bzw. Selektivitätswinkel.
Wie Fig. 8 zeigt, können Ultraschallwandler 22 mit orthogonal zur Oberfläche austretender Schallkeule, aber auch solche mit einer Schallkeule verwendet werden, welche unter einem Winkel α zur Oberfläche austritt.

Steuerungsseitig werden die Ultraschallwandler zyklisch oder azyklisch abwechselnd sowohl im Sender- als auch im Empfängermodus betrieben, wodurch eine weitgehende Störungsunterdrückung erreicht werden kann.

Die in der Fig. 9 zu erkennenden Blenden 19 mit Öffnungen 20 besitzen eine geringe Dicke vorzugsweise im Bereich von 0,2 bis 0,4 mm. Die Blenden 19 selbst können aus Edelstahl mit polierter, glatter Oberfläche gefertigt sein, so daß sich minimale Strömungsverluste aufgrund des gegebenen Strömungswiderstands einstellen. Die gewählte Struktur aus Blende 19 mit Öffnungen 20 zur Ausbildung der Strömungskanäle 15 gewährleistet in Verbindung mit der Anordnung der Ultraschallwandler 22 eine hohe Selektivität bei der Erfassung des Volumenstroms, wobei ein Selbstreinigungseffekt mit minimaler Verschmutzung auftritt.
Die aktive Wandlerfläche der Ultraschallsensorik ist relativ gering und bewegt sich beispielsweise im Bereich von 2 x 2 mm, wobei die Arbeitsfrequenz der Wandler zwischen 400 und 450 kHz liegt. Ziel ist es, eine recht hohe Schallenergie mit einem kleinen Austrittswinkel im genannten Frequenzbereich auszustrahlen, um die gewünschten Selektivitätseigenschaften für die Volumenstromerfassung zu gewährleisten.
Durch die Anordnung der Ultraschallwandler bzw. Ultraschallsensoren weit außerhalb der Mitte des Strömungskanals wird die Empfindlichkeit gegenüber dem zu messenden Volumenfluß erhöht und die Störanfälligkeit der Vorrichtung reduziert.

Eine weitere Verbesserung der Arbeitsweise der Volumenstromsensorik wird durch eine schmutz- und wasserabweisende Oberflächenbeschichtung bzw. eine Beschichtung der Impedanzanpassungsschicht der Ultraschallwandler 22 erreicht. Diese Beschichtung kann beispielsweise aus Polytetrafluorethylen oder einem ähnlichen Material bestehen. Bewvborzugt ist der Einsatz eines spritzgußfähigen Materials mit etwa 50 Gew.-% Glasfaserverstärkung auf Basis Copolyamid PA 6T/6I.

Die Ultraschallwandler 22 sind, wie gemäß Fig. 8 prinzipiell erkennbar, stoff- und/oder formschlüssig mit der umgebenden Oberfläche des Volumenstromsensorgehäuses bzw. einer dort angeordneten Zwischenwand verbunden.

Die Elektronik zur Ansteuerung der Ultraschallwandler ist bevorzugt Bestandteil der Signalvorverarbeitungs-Baugruppe, die wiederum im Bereich 16 (Fig. 7) befindlich ist.

Wie aus den Darstellungen ersichtlich, ist die Grundform des Volumenstromsensorgehäuses 3 im wesentlichen symmetrisch, wobei zwei Strömungskammern 14 mit entsprechenden Strömungskanälen 15 vorhanden sind, deren Öffnungen in verlängerter Richtung zur Lage der Öffnungen in der Atemgasmaske 1, bevorzugt im Bereich der Nüstern des Tieres gewählt sind.

Die Meßeinheit 17 kann über eine Antenne 23, die mit einem in der Meßeinheit befindlichen Telemetriemodul in Verbindung steht, einen Datenaustausch mit einer Basisstation durchführen, welche ebenfalls eine Telemetrieeinheit umfaßt. Neben dem uni- oder bidirektionalen Datenaustausch können auch Befehle, insbesondere zur Steuerung des Meßablaufs über diese Strecke geführt werden.

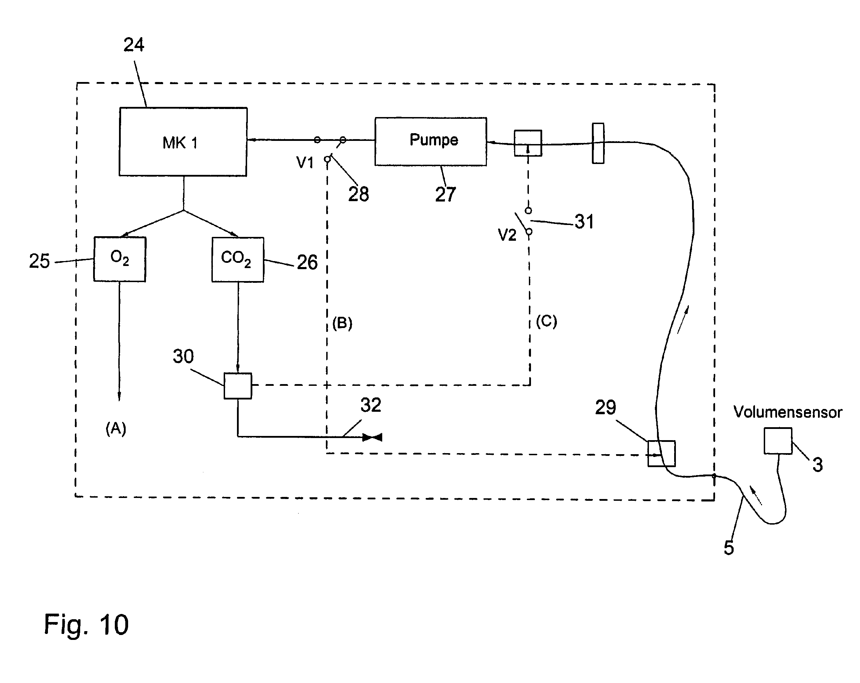

Mit Hilfe der Fig. 10 sei das Grundprinzip der Pneumatiksteuerung bei Anwendung der Mischkammer-Meßmethode erläutert.

In der Meßeinheit 17 ist demnach eine Mischkammer 24 befindlich, die mit einem O₂-Sensor 25 und einem CO₂-Sensor 26 in Verbindung steht.

Die Mischkammer 24 wird über eine Pumpe 27 mit Atemgas versorgt. Zwischen Pumpe 27 und dem Gaseingang der Mischkammer 24 ist ein erstes Ventil 28 (V1) geschaltet.

Ausgangsseitig steht das erste Ventil 28 mit einem Verteilerstutzen 29 in Verbindung, der auch zum Anschluß für den Gasabsaugschlauch 5 führt, welcher mit dem Pumpeneingang in Strömungsverbindung steht.

Die Pumpe 27 arbeitet kontinuierlich, und zwar während der Inspiration mit einem definierten Ruhewert und bei Expiration durch den Volumenstrom gesteuert, wodurch eine Beschleunigung aus dem Stillstand nicht erforderlich ist, was bei hoher Atemfrequenz erhebliche Vorteile bietet.

Um zu verhindern, daß in die Mischkammer 24 Umgebungsluft gelangt und daß sich der Absaugschlauch 5 mit Umgebungsluft füllt bzw. anreichert, ist während der Inspiration der Pneumatikkreis B wirksam. Das heißt, in diesem Fall bildet der Kreis B über das erste Ventil 28 in Verbindung mit dem Verteilerstutzen 29 einen Eingangs-Bypass.

Während der Expiration ist der Kreislauf A wirksam.

Beim Übergang von der Expiration zur Inspiration sorgt der Kreislauf C dafür, daß der Druckausgleich im Absaugschlauch 5 nicht durch die Umgebungsluft erfolgt, sondern Expirationsgas aus der vorangegangenen Expirationsphase einströmt. Der Kreis C ist nur während der kurzen Phase dieses erwähnten Druckausgleichs aktiviert. Zur Bildung des Kreislaufs C ist ein weiterer Verteilerstutzen 30 ausgangsseitig des CO₂-Sensors 26 befindlich und es ist ein zweites Ventil 31 (V2) vorhanden, das eine Verbindung zwischen dem Verteilerstutzen 30 und der Pumpeneingangsseite herstellt. Durch eine definierte Länge eines Schlauchstücks 32 ist gesichert, daß während der Phase des Druckausgleichs keine Umgebungsluft in den Kreis C einströmt.

Die beschriebene Lösung der Pneumatiksteuerung vermeidet den ansonsten gegebenen Nachteil, daß die Pumpe der höheren Atemfrequenz bei Tieren, die einer Belastung ausgesetzt sind, nicht mehr folgen kann. Weiterhin wird die Tatsache berücksichtigt, daß der im Absaugschlauch, welcher zwischen Volumenstromsensor und Pumpe angeordnet ist, entstehende Unterdruck aufgrund der höheren Atemfrequenz und der gegebenen größeren Länge nicht zu einer Meßwert-Verfälschung führt, weil üblicherweise unter unveränderter Anwendung des Standes der Technik nach Beendigung der Expirationsphase Umgebungsluft angesaugt werden kann, was es gerade zu verhindern gilt.

### Bezugszeichenliste

- 1: Atemgasmaske
- 2: Geschirr
- 3: Volumenstromsensorgehäuse
- 4: Befestigungsmittel
- 5: Absaugschlauch
- 6: elektrische Anschlüsse
- 7: Dichtkragen
- 8: Ventil
- 10: Luftpumpe
- 11: Gasaus- und -einlaßöffnung
- 13: Anschlußstutzen
- 14: Strömungskammer
- 15: Strömungskanäle
- 16: Bereich zur Anordnung der Signalvorverarbeitungs-Elektronik
- 17: Meßeinheit
- 18: Batterie-Pack oder Netzteil
- 19: Blende
- 20: Öffnung
- 21: gedachte Verbindungslinie
- 22: Ultraschallwandler
- 23: Antenne
- 24: Mischkammer
- 25: O₂-Sensor
- 26: CO₂-Sensor
- 27: Pumpe
- 28: erstes Ventil
- 29, 30: Verteilerstutzen
- 31: zweites Ventil
- 32: Schlauchstück

## Patentansprüche

1. Ergospirometriesystem für Tiere, insbesondere Pferde oder Kamele, umfassend eine trichterförmige, zylindrische oder halbkugelförmige Atemgasmaske (1), ein Gasvolumenstrom- oder Mengensensor, ein von der Atemgasmaske lösbares Volumenstrom sensorgehäuse (3), sowie eine Meßeinheit (17) mit Sensoren (25, 26) zur Bestimmung der CO₂/O₂-Konzentration im Atemgas nach dem Mischkammer- oder Breath-by-Breath-Prinzip und Anschluß- und/oder Signalübertragungsmittel zur Meßwert-Weiterverarbeitung, -Darstellung und/oder -Analyse in einer Basisstation,
wobei
an der Atemgasmaske Mittel (4) zum Befestigen des von der Maske lösbaren Volumenstromsensorgehäuses (3) vorgesehen sind, das Volumenstromsensorgehäuse mindestens eine Strömungskammer (14) aufweist, wobei die mindestens eine Strömungskammer jeweils Gaseinlaß- bzw. Gasauslaßöffnungen umfassen, welche einerseits in Strömungskontakt mit zugehörigen Öffnungen der Maske und andererseits zur Umgebung stehen,
weiterhin innerhalb der Strömungskammern durch in Strömungsrichtung verlaufende, parallel und beabstandet angeordnete Blenden Strömungskanäle gebildet sind, wobei die Blenden (19) versetzt ausgebildete Aussparungen oder Öffnungen vorgebbaren Querschnitts besitzen, deren Schwer- oder Mittelpunkte im wesentlichen auf einer gedachten Verbindungslinie liegen und wobei weiterhin an den Endpunkten der gedachten Linie Ultraschallwandler (22) zur Bestimmung des Volumenstroms befindlich sind, wobei die Schallkeule der Wandler im wesentlichen entlang der Verbindungslinie (21) oder auf dieser verläuft.

2. Ergospirometriesystem nach Anspruch 1,
**dadurch gekennzeichnet, daß**
die Aussparungen oder Öffnungen in den Blenden eine kreisrunde oder elliptische Querschnittsform aufweisen, wobei durch die Blendenform und versetzte Anordnung unerwünschte Schallreflexionen unterdrückbar sind.

3. Ergospirometriesystem nach Anspruch 1 oder 2,
**dadurch gekennzeichnet, daß**
die Atemgasmaske derart abgedichtet am Kopf des Tieres anbringbar ist, daß der Atemgasstrom ausschließlich zu oder über den im Volumenstromsensorgehäuse vorgesehenen Strömungskammern führt, wobei zwischen dem Volumenstromsensorgehäuse und der Atemgasmaske weitere Dichtmittel oder Dichtflächen ausgebildet sind und wobei die Atemgasmaske vorzugsweise einen integralen, aufblasbaren umlaufenden Dichtkragen (7) aufweist.

4. Ergospirometriesystem nach einem der vorangegangenen Ansprüche,
**dadurch gekennzeichnet, daß**
die eingesetzten Ultraschallwandler eine gering dimensionierte aktive Wandlerfläche mit einer Strahlungskeule kleinen Öffnungswinkels aufweisen.

5. Ergospirometriesystem nach Anspruch 4,
**dadurch gekennzeichnet, daß**
die Oberfläche der Wandler mit einer schmutz- und wasserabweisenden Beschichtung versehen ist.

6. Ergospirometriesystem nach einem der vorangegangenen Ansprüche,
**dadurch gekennzeichnet, daß**
die Wandler mit dem umgebenden Gehäuseteil oder der Gehäusefläche des Volumenstromsensorgehäuses stoff- und/oder formschlüssig verbunden sind.

7. Ergospirometriesystem nach einem der vorangegangenen Ansprüche,
**dadurch gekennzeichnet, daß**
die Arbeitsfrequenz der Wandler im Bereich von im wesentlichen 350 bis 500 kHz, vorzugsweise 400 bis 450 kHz liegt.

8. Ergospirometriesystem nach einem der vorangegangenen Ansprüche,
**dadurch gekennzeichnet, daß**
die Blenden eine geringe, strömungsoptimale Dicke aufweisen, wobei die Anordnung der Blenden der Ausbildung einer gleichgerichteten turbulenten, aber weitgehend wirbelfreien Strömung innerhalb der Strömungskammern dient.

9. Ergospirometriesystem nach Anspruch 8,
**dadurch gekennzeichnet, daß**
die Dicke der Blenden im Bereich von im wesentlichen 0,15 bis 0,45 mm liegt.

10. Ergospirometriesystem nach einem der vorangegangenen Ansprüche,
**dadurch gekennzeichnet, daß**
im Volumenstromsensorgehäuse eine Signalvorverarbeitungs-Elektronik (16) zur Ansteuerung der Wandler und zur Bestimmung des Volumenflusses angeordnet ist, um die derartig vorverarbeiteten elektrischen Signale zur Meßeinheit (17) zu übertragen.

11. Ergospirometriesystem nach einem der vorangegangenen Ansprüche,
**dadurch gekennzeichnet, daß**
am Volumenstromsensorgehäuse ein Gasabsaug-Anschlußstutzen (13) zur Herstellung einer Verbindung zum Gastransport in die Meßeinheit vorgesehen ist.

12. Ergospirometriesystem nach einem der vorangegangenen Ansprüche,
**dadurch gekennzeichnet, daß**
das Volumenstromsensorgehäuse eine im wesentlichen symmetrische Grundform mit zwei Strömungskammern (14) besitzt, wobei die Strömungskammern sich in verlängerter Richtung der Öffnungen in der Atemgasmaske, bevorzugt im Bereich der Nüstern des Tieres, erstrecken.

13. Ergospirometriesystem nach Anspruch 12,
**dadurch gekennzeichnet, daß**
in jeder Strömungskammer Ultraschallwandler angeordnet sind,
wobei durch die zugehörige Auswerteelektronik die Gasvolumina bzw. der Volumenfluß je Kammer und ein Summenvolumenfluß ermittelbar ist.

14. Ergospirometriesystem nach einem der vorangegangenen Ansprüche,
**dadurch gekennzeichnet, daß**
die Meßeinheit ein Telemetriemodul und die Basisstation eine Telemetrieeinheit zum Aufbau einer uni- oder bidirektionalen Daten- und/oder Befehlsübertragungsstrecke aufweisen.

15. Ergospirometriesystem nach Anspruch 14,
**dadurch gekennzeichnet, daß**
die Telemetrieeinheit der Basisstation eine automatische Frequenzwahleinrichtung aufweist, um nach Scannen des Frequenzbands und/oder einer Testdaten-Übertragungsprozedur eine Telemetriefrequenz mit erwarteter optimaler Übertragungsqualität festzulegen.

16. Ergospirometriesystem nach einem der vorangegangenen Ansprüche,
**gekennzeichnet durch**
einen Speicher zur Datensicherung in der Meßeinheit, wobei im Speicher Datensätze im definierten Format aus atemzugsbezogenen Meßdaten für jeden Atemzug und/oder für sämtliche oder ausgewählte Meßgrößen die kompletten Meßdatenverläufe über wählbare Intervalle zu wählbaren Startzeiten abgelegt werden.

17. Ergospirometriesystem nach einem der vorangegangenen Ansprüche,
**dadurch gekennzeichnet, daß**
die Meßeinheit Sensoren zur Bestimmung der Umgebungstemperatur und/oder der Atemgasfeuchte und/oder weiterer relevanter Größen, wie z.B. Differenzdruckwerte aufweist, wobei diese Sensorwerte zur Korrektur der Atemgassensorwerte herangezogen werden.

18. Ergospirometriesystem nach einem der vorangegangenen Ansprüche,
**dadurch gekennzeichnet, daß**
die Meßeinheit am Körper des Tieres oder einer das Tier führenden Person applizierbar ist.

19. Ergospirometriesystem nach Anspruch 11,
**dadurch gekennzeichnet, daß**
die Meßeinheit eine Mischkammer mit einer Pneumatiksteuerung aufweist, wobei an der Mischkammer die CO₂/O₂-Sensoren, mit dieser in Strömungsverbindung stehend, angeschlossen sind, weiterhin eine Pumpe (27) mit einem Anschluß für den Absaugschlauch vorgesehen ist, wobei der Absaugschlauch zum Volumenstromsensorgehäuse führt und wobei mittels der Pneumatiksteuerung zwei Strömungskreisläufe für Expiration und Inspiration schaltbar sind.

20. Ergospirometriesystem nach Anspruch 19,
**dadurch gekennzeichnet, daß**
die Pumpe im quasi Dauerbetrieb arbeitet, wobei während der Inspiration die Pumpe mit einem Ruhewert einen Eingangs-Strömungskreislauf versorgt, wodurch ein Eindringen von Umgebungsluft in die Mischkammer und/oder den Absaugschlauch verhinderbar ist, und daß bei Expiration die Pumpe volumenstromgesteuert die Mischkammer mit Atemgas versorgt, wobei während der Übergangsphase von Expiration zu Inspiration der Druckausgleich über eine durch die Pneumatiksteuerung zeitweise geschaltete Verbindung zwischen Mischkammer und Pumpeneingang erfolgt, wodurch das Eindringen von Umgebungsluft in den Absaugschlauch vermeidbar ist.

## Claims

1. An ergospirometry system for animals, in particular horses or camels, comprising a funnel-shaped, cylindrical or semispherical breathing gas mask (1), a gas volume flow rate or quantity sensor, a volume flow rate sensor housing (3) which may be separated from the breathing gas mask, as well as a measuring unit (17) with sensors (25, 26) for the determination of the CO₂/O₂ concentration in the breathing gas in accordance with the mixing chamber or breath-by-breath principle, and connection and/or signal transmission means for the further processing of the measured values, their representation and/or analysis in a base station,
wherein means (4) are provided at the breathing gas mask for fastening the volume flow rate sensor housing (3) which may be separated from the mask, the volume flow rate sensor housing comprises at least one flow chamber (14), with the at least one flow chamber comprising gas inlet or gas outlet openings, respectively, which, on the one hand, are in flow contact with associated openings of the mask and, on the other hand, with the environment,
further with flow channels being formed within the flow chambers by diaphragms extending in the flow direction, arranged parallel and spaced from each other, with the diaphragms (19) having offset recesses or openings with specifiable cross-section, whose centres of gravity or centres essentially lie on an imaginary connecting line, and further with ultrasonic transducers (22) being located at the end points of the imaginary line for the determination of the volume flow rate, with the sound lobe of the transducers extending essentially along the connecting line (21) or on it.

2. The ergospirometry system according to Claim 1,
**characterised in that**
the recesses or openings in the diaphragms comprise a circular or elliptical cross-sectional shape, with undesired sound reflections being suppressible by the diaphragm shape and the offset arrangement.

3. The ergospirometry system according to Claim 1 or 2,
**characterised in that**
the breathing gas mask is adapted to be fitted at the animal's head in a sealed manner so that the breathing gas stream exclusively leads to or via the flow chambers provided in the volume flow rate sensor housing, with further sealing means or sealing areas being formed between the volume flow rate sensor housing and the breathing gas mask, and with the breathing gas mask preferably comprising an integral, inflatable, circumferential sealing collar (7).

4. The ergospirometry system according to one of the previous claims,
**characterised in that**
the employed ultrasonic transducers comprise an active transducer area of small dimensions with a radiation lobe of a small opening angle.

5. The ergospirometry system according to Claim 4,
**characterised in that**
the surface of the transducers is provided with a dirt and water repellent coating.

6. The ergospirometry system according to one of the previous claims,
**characterised in that**
the transducers are connected with the surrounding housing part or the housing area of the volume flow rate sensor housing in an adhesively connecting and/or positive manner.

7. The ergospirometry system according to one of the previous claims,
**characterised in that**
the working frequency of the transducers is within essentially 350 to 500 kHz, preferably within 400 to 450 kHz.

8. The ergospirometry system according to one of the previous claims,
**characterised in that**
the diaphragms comprise a small thickness for an optimum flow, with the arrangement of the diaphragms serving to generate a rectified turbulent, but essentially irrotational flow within the flow chambers.

9. The ergospirometry system according to Claim 8,
**characterised in that**
the thickness of the diaphragms ranges from essentially 0.15 to 0.45 mm.

10. The ergospirometry system according to one of the previous claims,
**characterised in that**
a signal preprocessing electronic circuit (16) for driving the transducers and for determining the volume flow rate is arranged in the volume flow rate sensor housing in order to transmit the electric signals so preprocessed to the measuring unit (17).

11. The ergospirometry system according to one of the previous claims,
**characterised in that**
a gas evacuating connecting pipe (13) is provided for establishing a connection to the gas transport into the measuring unit.

12. The ergospirometry system according to Claim 8,
**characterised in that**
the volume flow rate sensor housing has an essentially symmetric basic shape with two flow chambers (14), with the flow chambers extending in the extended direction of the openings in the breathing gas mask, preferably in the region of the animal's nostrils.

13. The ergospirometry system according to Claim 12,
**characterised in that**
ultrasonic transducers are arranged in each flow chamber, with the gas volumes or the volume flow rate, respectively, per chamber and a total volume flow rate being able to be determined by the associated evaluation electronics.

14. The ergospirometry system according to one of the previous claims,
**characterised in that**
the measuring unit comprises a telemetry module and the base station comprises a telemetry unit for establishing a unidirectional or bidirectional data and/or command transmission link.

15. The ergospirometry system according to Claim 14,
**characterised in that**
the telemetry unit of the base station comprises an automatic frequency selection device in order to specify a telemetry frequency with expected optimum transmission quality after sampling the frequency band and/or after a test data transmission procedure.

16. The ergospirometry system according to one of the previous claims,
**characterised by**
a storage for data saving in the measuring unit, with data records in a defined format from breathing-related measured data for each breath and/or for all or for selected measured quantities for the complete measured data distributions over selectable intervals to selectable starting times being stored.

17. The ergospirometry system according to one of the previous claims,
**characterised in that**
the measuring unit comprises sensors for determining the ambient temperature and/or the breathing gas humidity, and/or further relevant quantities, such as e. g. differential pressure values, with these sensor values being used for the correction of the breathing gas sensor values.

18. The ergospirometry system according to one of the previous claims,
**characterised in that**
the measuring unit may be applied to the animal's body or to a person leading the animal.

19. The ergospirometry system according to Claim 11,
**characterised in that**
the measuring unit comprises a mixing chamber with a pneumatic controlling means, with the CO₂/O₂ sensors being connected in flow communication with the mixing chamber, further a pump (27) is provided with a connection for the evacuation hose, with the evacuation hose leading to the volume flow rate sensor housing and with two flow circuits for expiration and inspiration being connectable by means of the pneumatic controlling means.

20. The ergospirometry system according to Claim 19,
**characterised in that**
the pump works in a quasi permanent operation mode, with the pump providing an input flow circuit with a quiescent value during inspiration whereby an ingress of ambient air into the mixing chamber and/or the evacuation hose can be prevented, and **in that** the pump supplies the mixing chamber with breathing gas during expiration in a volume flow controlled manner, with the pressure compensation being effected during the transition phase from expiration to inspiration via a connection between mixing chamber and pump inlet which is intermittently selected by the pneumatic controlling means, so that the ingress of ambient air into the evacuation hose can be prevented.

## Revendications

1. Système d'ergospirométrie pour animaux, en particulier pour chevaux ou chameaux, comprenant un masque à gaz respiratoire (1) en forme d'entonnoir, cylindrique ou en forme d'hémisphère, un capteur de débit volumétrique ou de quantité de gaz, un boîtier de capteur de débit volumétrique (3) détachable du masque à gaz respiratoire, ainsi qu'une unité de mesure (17) avec des capteurs (25, 26) pour déterminer la concentration en CO₂/O₂ dans le gaz respiratoire d'après le principe de chambre de mélange ou le principe breath-by-breath et des moyens de raccordement et/ou de transmission de signaux pour le traitement, la représentation et/ou l'analyse de valeurs mesurées dans une station de base,
dans lequel des moyens (4) de fixation du boîtier de capteur de débit volumétrique (3) détachable du masque sont prévus sur le masque à gaz respiratoire, le boîtier de capteur de débit volumétrique présente au moins une chambre d'écoulement (14), ladite au moins une chambre d'écoulement comprenant des ouvertures d'admission de gaz et des ouvertures de sortie de gaz respectives qui sont en contact d'écoulement d'une part avec des ouvertures associées du masque et d'autre part avec l'environnement, des canaux d'écoulement sont en outre formés à l'intérieur des chambres d'écoulement par des obturateurs s'étendant en direction d'écoulement et agencés en parallèle et à distance, les obturateurs (19) possédant des évidements ou ouvertures de section transversale prédéterminable réalisés en décalage, dont les centres de gravités ou points centraux sont sensiblement sur une ligne de liaison imaginaire et dans lequel en outre des transducteurs d'ultrasons (22) pour déterminer le débit volumétrique se trouvent aux points d'extrémité de la ligne imaginaire, le lobe acoustique du transducteur s'étendant sensiblement le long de la ligne de liaison (21) ou sur celle-ci.

2. Système d'ergospirométrie selon la revendication 1, **caractérisé en ce que** les évidements ou ouvertures dans les obturateurs présentent une forme de section transversale circulaire ou elliptique, des réflexions acoustiques indésirables pouvant être éliminées par la forme de l'obturateur et par l'agencement en décalage.

3. Système d'ergospirométrie selon la revendication 1 ou 2,
**caractérisé en ce que**
le masque à gaz respiratoire peut être appliqué de manière étanche sur la tête de l'animal de telle sorte que le flux de gaz respiratoire mène exclusivement vers ou au-dessus des chambres d'écoulement prévues dans le boîtier de capteur de débit volumétrique, et entre le boîtier de capteur de débit volumétrique et le masque à gaz respiratoire sont réalisés d'autres moyens d'étanchéité ou surfaces d'étanchéité et le masque à gaz respiratoire présente de préférence une collerette d'étanchéité (7) intégrale périphérique gonflable.

4. Système d'ergospirométrie selon l'une des revendications précédentes,
**caractérisé en ce que**
les transducteurs d'ultrasons utilisés présentent une surface de transducteur active à faibles dimensions avec un lobe de rayonnement à petit angle d'ouverture.

5. Système d'ergospirométrie selon la revendication 4,
**caractérisé en ce que**
la surface des transducteurs est pourvue d'un revêtement anti-salissant et hydrofuge.

6. Système d'ergospirométrie selon l'une des revendications précédentes,
**caractérisé en ce que**
les transducteurs sont reliés par coopération de matières ou de formes à la partie de boîtier environnante ou à la surface du boîtier de capteur de débit volumétrique.

7. Système d'ergospirométrie selon l'une des revendications précédentes,
**caractérisé en ce que**
la fréquence de travail des transducteurs est dans la plage de sensiblement 350 à 500 kHz, de préférence de 400 à 450 kHz.

8. Système d'ergospirométrie selon l'une des revendications précédentes,
**caractérisé en ce que**
les obturateurs présentent une épaisseur faible optimale à l'écoulement, l'agencement des obturateurs servant à un écoulement turbulent agissant dans le même sens, mais en grande partie laminaire, dans les chambres d'écoulement.

9. Système d'ergospirométrie selon la revendication 8,
**caractérisé en ce que**
l'épaisseur des obturateurs est dans la plage de sensiblement 0,15 à 0,45 mm.

10. Système d'ergospirométrie selon l'une des revendications précédentes,
**caractérisé en ce que**
dans le boîtier de capteur de débit volumétrique est agencé un système électronique (16) de traitement préalable de signal pour piloter les transducteurs et pour déterminer le débit volumétrique pour transmettre des signaux électriques ainsi préalablement traités à l'unité de mesure (17).

11. Système d'ergospirométrie selon l'une des revendications précédentes,
**caractérisé en ce que**
sur le boîtier de capteur de débit volumétrique est prévue une tubulure de raccordement d'aspiration de gaz (13) pour réaliser une liaison afin de transporter le gaz dans l'unité de mesure.

12. Système d'ergospirométrie selon l'une des revendications précédentes,
**caractérisé en ce que**
le boîtier de capteur de débit volumétrique possède une forme de base sensiblement symétrique avec deux chambres d'écoulement (14), les chambres d'écoulement s'étendant en direction prolongée des ouvertures dans le masque à gaz respiratoire, de préférence dans la région des naseaux de l'animal.

13. Système d'ergospirométrie selon la revendication 12,
**caractérisé en ce que**
dans chaque chambre d'écoulement sont agencés des transducteurs à ultrasons, et au moyen du système électronique d'évaluation associé, on peut déterminer les volumes de gaz ou le débit volumétrique par chambre et un débit volumétrique total.

14. Système d'ergospirométrie selon l'une des revendications précédentes,
**caractérisé en ce que**
l'unité de mesure présente un module de télémétrie, et la station de base présente une unité de télémétrie pour établir une liaison de transmission de données et/ou d'instructions unidirectionnelle ou bidirectionnelle.

15. Système d'ergospirométrie selon la revendication 14,
**caractérisé en ce que**
l'unité de télémétrie de la station de base présente un moyen de sélection de fréquence automatique pour déterminer une fréquence de télémétrie avec qualité de transmission optimale attendue après le scannage de la bande de fréquence et/ou après une procédure de transmission de données d'essai.

16. Système d'ergospirométrie selon l'une des revendications précédentes,
**caractérisé par**
une mémoire pour sauvegarder les données dans l'unité de mesure, et dans la mémoire sont stockés des jeux de données dans le format défini, à partir de données de mesure se rapportant à la respiration pour chaque cycle respiratoire et/ou pour toutes les grandeurs mesurées ou pour les grandeurs mesurées sélectionnées sont stockés les déroulements complets des données de mesure via des intervalles sélectionnables à des temps de démarrage sélectionnables.

17. Système d'ergospirométrie selon l'une des revendications précédentes,
**caractérisé en ce que**
l'unité de mesure présente des capteurs pour déterminer la température ambiante et/ou l'humidité du gaz respiratoire et/ou d'autres grandeurs significatives telles que les valeurs de pression différentielle, ces valeurs de capteur étant mises à contribution pour corriger les valeurs de capteur de gaz respiratoire.

18. Système d'ergospirométrie selon l'une des revendications précédentes,
**caractérisé en ce que**
l'unité de mesure peut être appliquée sur le corps de l'animal ou sur une personne guidant l'animal.

19. Système d'ergospirométrie selon la revendication 11
**caractérisé en ce que**
l'unité de mesure présente une chambre de mélange avec une commande pneumatique, et à la chambre de mélange sont branchés les capteurs de CO₂/O₂ qui sont en liaison d'écoulement avec celle-ci, et par ailleurs est prévue une pompe (27) avec un raccordement pour un tuyau d'aspiration, le tuyau d'aspiration menant au boîtier de capteur de débit volumétrique et deux circuits d'écoulement pour l'expiration et pour l'inspiration pouvant être commutés au moyen de la commande pneumatique.

20. Système d'ergospirométrie selon la revendication 19,
**caractérisé en ce que**
la pompe fonctionne en service quasiment permanent, et pendant l'inspiration, la pompe alimente un circuit d'écoulement d'admission avec une valeur au repos, ce qui permet d'empêcher que de l'air ambiant pénètre dans la chambre de mélange et/ou dans le tuyau d'aspiration, et **en ce que** lors de l'expiration, la pompe alimente par commande de débit volumétrique la chambre de mélange en gaz respiratoire, et pendant la phase de transition entre l'expiration et l'inspiration, la compensation de pression a lieu via une liaison commutée temporairement par la commande pneumatique entre la chambre de mélange et l'entrée de pompe, ce qui permet d'empêcher que l'air ambiant pénètre dans le tuyau d'aspiration.
